# EUROPEAN PATENT APPLICATION

(11) **EP 2 202 518 A1**
(43) Date of publication of application: **30.06.2010**
(21) Application number: 08022152.6
(22) Date of filing: 19.12.2008
(51) Int. Cl.: G01N 33/50

(54) **In vivo assay for quantifying clostridial neurotoxin activity**

(71) Applicant: Merz Pharma GmbH & Co. KGaA, 60318 Frankfurt (DE)
(72) Inventor: Wilk, Thomas, Dr., 67117 Limburgerhof (DE); Taylor, Harold, Dr., 60435 Frankfurt am Main (DE); Eisele, Karl-Heinz, Dr., 70794 Filderstadt (DE)
(74) Representative: Ricker, Mathias

(57) **Abstract**

Novel methods for determining the unknown biological activity of a clostridial neurotoxin in a sample with respect to the known biological activity of a clostridial neurotoxin in a reference sample, comprising the step of comparing the biological activity of a clostridial neurotoxin preparation with the biological activity of a standard preparation of a reference clostridial neurotoxin in certain eumetazoan animals.

## Description

### FIELD OF THE INVENTION

This invention relates to novel methods for determining the unknown biological activity of a clostridial neurotoxin in a sample with respect to the known biological activity of a clostridial neurotoxin in a reference sample. The method comprises the step of comparing the biological activity of a clostridial neurotoxin preparation with the biological activity of a standard preparation of a reference clostridial neurotoxin in certain eumetazoan animals.

### BACKGROUND OF THE INVENTION

In recent years, botulinum neurotoxins have become the standard agent in the treatment of focal dystonias and spastic indications. Treatment of patients generally involves injection of the neurotoxin into affected muscle tissue, bringing the agent near the neuromuscular end plate, i.e. close to the cellular receptor mediating its uptake into the nerve cell controlling said affected muscle. Various degrees of neurotoxin spread have been observed. This spread is thought to correlate with the injected amounts and the particular preparation of neurotoxin injected. Resulting from the spread, systematic side effects caused by the inhibition of acetylcholine release, may be observed at nearby muscle tissue. The incidents of unintended paralysis of untreated muscles can largely be avoided by reducing the injected doses to the therapeutically relevant level. Overdosing may also be a problem with regard to the patients' immune system, as the injected neurotoxin may trigger the formation of neutralizing antibodies. If this occurs, the neurotoxin will be inactivated without being able to relieve the involuntary muscle activity.

Differences in the dose equivalents or variations in the determined activity of preparations such as available sales products or batches produced during the manufacturing process, commonly a fermentation process, pose an increased risk for patients through possible side effects and the development of immunity. Therefore, it is of crucial importance to determine the biological activity of a clostridial neurotoxin contained in said sales products or production batches reliably (i.e. without significant variation) and as accurately as possible, in order to adjust the neurotoxin concentration to a reliable effective dose for the benefit of the patient. This may also serve as an incentive to the manufacturers to offer formulations allowing optimum exploitation of biological activity for different therapeutic purposes.

At present, the botulinum neurotoxin testing is predominantly performed using the mouse LD₅₀ assay developed more than 40 year ago (see Boroff and Fleck, J. Bacteriol. 92 (1966) 1580-1581), which is accepted for potency testing by United States and European regulatory agencies. This assay involves dosing mice with dilutions of the sample of botulinum neurotoxin being tested and calculating the dilution at which 50% of the mice would be expected to die. Since this bioassay requires up to 100 mice for testing a single sample, and takes up to four days to generate results, there is a large need for alternative methods that are faster and more accurate, and/or reduce, cause less pain, distress, and/or replace use of animals such as mice. Any such alternative method, in order to be acceptable to regulatory agencies for the determination of potency, must be suitable for the intended purpose of the product in question and must be validated for sensitivity, specificity, reproducibility and robustness. For botulinum neurotoxin testing, a suitable potency assay must be used to determine the dose of the final product or to compare the relative activities of different lots. Because botulinum neurotoxin activity is dependent upon three functional domains within the protein molecule, an acceptable potency assay must account for the activity of all domains. For a discussion of issues relating to the unsolved problem of mouse LD₅₀ assay replacement, see "Report on the ICCVAM-NICEATM/ECVAM Scientific Workshop on Alternative Methods to Refine, Reduce or Replace the Mouse LD50 Assay for Botulinum neurotoxin Testing", NIH Publication Number 08-6416, February 2008.

Crisley, Appl Microbiol. 8 (1960) 282-285, describes the development of an toxicity assay for supernatants from frozen stock cultures of *Clostridium botulinum* Type A in goldfish (*Carassius auratus*). Despite being known since about 1920, such an assay using goldfish has neither been used, nor suggested to be used, for determining the potency of clostridial neurotoxin preparations for pharmaceutical and/or aesthetic use.

Fenicia et al. (in NIH Publication Number 08-6416, *loc*. *cit.*) show results from preliminary experiments in microcrustacean *Daphnia magna*, demonstrating that *Daphnia magna* are sensitive to botulinum neurotoxins, including botulinum neurotoxin serotype A.

In addition to in vivo assays in animals, several approaches have been pursued to identifiy assay systems using vertebrate muscular tissue preparations, e.g. comprising the hind limb muscle or the hind limb extensor digitorum longus muscle of mice and rats, the plantar muscles of the hind paw e.g. of the mouse, the phrenic nerve-hemidiaphragm e.g. of the rat or mouse, the levator auris longus muscle e.g. of the mouse and rat, the frog neuromuscular junction, the biventer cervic muscle of chicks, the rib muscles or brain tissue e.g. of the mouse and rat or the electric organ of the sea ray.

Furthermore, the effect of clostridial neuroxins on the neuronal tissue of *Aplysia californica* has been described (see Mochida et al., Proc. Natl. Acad. Sci. USA 87 (1990) 7844-7848; Poulain et al., Proc. Natl. Acad. Sci. USA 85 (1988) 4090-4094).

The above referenced prior art methods, however, have not resulted in a method certified by regulatory authorities. Therefore, the out-of-time mouse killing assay must still be performed, and the need still exists to identify alternative assays.

### OBJECTS OF THE INVENTION

It was an object of the invention to improve the methods of the prior art and to develop reliable and accurate methods for determining the unknown biological activity of clostridial neurotoxins in a sample, particularly for pharmaceutical and/or aesthetic use, so that such methods might be used for regulatory purposes. Such an improved method would also serve to satisfy the great need for a safe and effective administration.

### SUMMARY OF THE INVENTION

Surprisingly it has been found that the methods for determining the unknown concentration of botulinum neurotoxins in a sample for clincial and/or aesthetic use can be developed in non-mammalian species.

In one aspect, the present invention relates to a method for determining the relative biological activity of a clostridial neurotoxin preparation, comprising the step of comparing the biological activity of said clostridial neurotoxin preparation with the biological activity of a standard preparation of a reference clostridial neurotoxin in an eumetazoan animal, provided that said animal is not a mammalian species.

In another aspect, the present invention also relates to an in vivo method for determining the biological activity of a clostridial neurotoxin preparation, comprising the steps of (a) contacting an animal with a sample comprising a clostridial neurotoxin preparation; and (b) comparing the biological effect of the sample with the biological effect of a reference sample wherein said animal is a Mollusca.

### DETAILED DESCRIPTION OF THE INVENTION

The present invention may be understood more readily by reference to the following detailed description of the invention and the examples included therein.

In the context of the present invention, the term "clostridial neurotoxin" refers to a natural neurotoxin obtainable from bacteria of the class Clostridia, including *Clostridium tetani* and *Clostridium botulinum*, or to a neurotoxin obtainable from alternative sources, including from recombinant expression or from genetic or chemical modification. Particularly, the clostridial neurotoxins have endopeptidase activity. Said neurotoxin may be obtained from a clostridial cell or by expression of the neurotoxin in a heterologous cell such as *E. coli.*

In the context of the present invention, the term "clostridial neurotoxin preparation" refers to a composition comprising a clostridial neurotoxin, including, but not limited to, the raw material obtained from a fermentation process (supernatant, composition after cell lysis), a fraction comprising a clostridial neurotoxin obtained from separating the ingredients of such a raw material in a purification process, an isolated and essentially pure clostridial neurotoxin, and a formulation for pharmaceutical and/or aesthetic use comprising a clostridial neurotoxin and additionally pharmaceutically acceptable solvents and/or excipients.

In the context of the present invention, the term "standard preparation of a reference clostridial neurotoxin" refers to a preparation comprising a clostridial neurotoxin, where the biological activity of a given amount of such standard preparation is known. The biological activity of such standard preparation may, for example, be determined by the mouse LD₅₀ assay described above. The LD₅₀ value defines the quantity of neurotoxin at which 50% of a mouse population is killed if said quantity is applied to the mice of said mouse population. The LD₅₀ value defines the so-called "unit" that is the commonly accepted unit used to define a quantity of a clostridial neurotoxin contained in a sample. The method for determining said value is known to the person skilled in the art. The method is documented in the European Pharmacopoeia (6^{th} edition; available from European Directorate for the Quality of Medicines & Healthcare (EDQM), 7 allée Kastner, CS 30026, F-67081 Strasbourg, France).

In the context of the present invention, the term "eumetazoan animal" refers to an animal belonging to the subkingdom "Eumetazoa" of the Kingdom "Animalia" being characterized by the presence of tissue. In particular, such animals comprise a nervous system. The term further includes animals in all development stages that already exhibit tissue differentiation, including the presence of a nervous system.

The animals used in the methods of the present invention generally express at least one receptor for cellular uptake of clostridial neurotoxin (for example, corresponding to human SV2 for botulinum neurotoxin serotype A), and at least one intracellular target for such clostridial neurotoxin (for example, corresponding to human SNAP25 for botulinum neurotoxin serotype A). Said target is a protein, which is involved in the formation of a functional SNARE complex. In certain such embodiments, the animal comprises at least one gene that is an ortholog of a human gene encoding a receptor for cellular uptake of such clostridial neurotoxin (for example, human SV2 for botulinum neurotoxin serotype A), and/or at least one gene that is an orthologue of a human gene encoding an intracellular target of such clostridial neurotoxin (for example, human SNAP25 for botulinum neurotoxin serotype A). In one embodiment, said target is inactivated by interaction with the neurotoxin, in another embodiment, said target is inactivated by proteolytic cleavage. Alternatively, said animal is a transgenic animal expressing a suitable target gene from a heterologous source, for example, a human SNARE protein such as SNAP25 or VAMP. Using a transgenic animal may be necessary in certain instances, in particular those where the species-specific SNARE proteins are no substrate for the neurotoxin.

In one embodiment of the method according to the first aspect of the invention, said method further comprises the step of administering to said animal, or otherwise exposing said animal with, at least one sample having a defined amount of said clostridial neurotoxin preparation.

In the context of the present invention, the term "administering to said animal ... at least one sample" refers to a step where such sample is either essentially directly applied to a part of such animal where the biological activity is supposed to occur (for example, by intramuscular injection), or is systemically applied to the animal (for example, by subcutaneous, intraperitoneal or intravenous injection, or by feeding the animal), and the term "otherwise exposing said animal with ... at least one sample" refers to any other application, where such animal is brought in contact with such sample (for example, by adding such sample to the aqueous environment of an animal living in such an environment).

In certain embodiments of the method according to the first aspect of the invention, said animal is an animal belonging to (i) a phylum taken from the list of: Acanthocephala, Acoelomorpha, Annelida, Arthropoda, Brachiopoda, Bryozoa, Chaetognatha, Cnidaria, Ctenophora, Cycliophora, Echinodermata, Echiura, Entoprocta, Gastrotricha, Gnathostomulida, Hemichordata, Kinorhyncha, Loricifera, Micrognathozoa, Mollusca, Nematoda, Nematomorpha, Nemertea, Onychophora, Orthonectida, Phoronida, Platyhelminthes, Priapulida, Rhombozoa, Rotifera, Sipuncula, Tardigrada, and Xenoturbellida; (ii) a subphylum taken from the list of: Tunicata, and Cephalocordata; or (iii) a class taken from the list of: Myxinomorphi, Petromyzontomorphi, Chondrichthiomorphi, Amphibia, Reptilia, and Aves.

In another embodiment, said animal is an animal belonging (i) to the phylum Mollusca,; (ii) to a subphylum taken from the list of: Tunicata and Hexapoda (at all stages of development, including nymphae, larvae, pupae and imagines), or (iii) to a class taken from the list of: Amphibia (at all stages of development, including tadpoles and adults) and Reptilia,

In another embodiment, said Mollusca is selected from the group consisting of Aplacophora, Bivalvia, Caudofoveata, Cephalopoda, Gastropoda, Monoplacophora, Polyplacophora and Scaphopoda.

In another embodiment, said Bivalvia is selected from the group consisting of Anomalosdesmata, Cryptodonta, Heterodonta, Paleoheterodonta, Palaeotaxodonta, Pteriomorphia.

In another embodiment, said Heterodonta is Myoida or Veneroida.

In another embodiment, said Paleoheterodonta is selected from the group consisting of Lametilidae, Malletiidae, Neilonellidae, Nuculanidae, Nuculidae, Praenuculidae, Pristiglomidae, Siliculidae, Tindariidae and Yoldiidae.

In another embodiment, said said Pteriomorphia is selected from the group consisting of Arcoida, Limoida, Mytiloida, Ostreoida and Pterioida.

In certain embodiments of the first aspect of the present invention, the biological activity is the inhibition of muscle tissue activity.

In the context of the present invention, the term "inhibition of muscle tissue activity" refers to the property of clostridial neurotoxins to inhibit activation of muscle tissue by blocking the release of neurotransmitters from presynaptic vesicles.

In certain embodiments of the present invention, said inhibition is determined by directly determining the activity of muscle tissue.

In the context of the present invention, the term "muscle tissue" refers to any muscle or part of a muscle the activity of which is accessible for activity measurements, including both smooth and striated muscles, if available, and individual or multiple muscle fibers, including whole muscle bundles. The term "directly determining the activity of said muscle tissue" refers to methods where parameters relating to the activity of a muscle tissue are directly read out.

In particular embodiments, said activity is determined by determining one or more of the following parameters: time to paralysis of said muscle tissue, contraction rate of said muscle tissue, contraction distance of said muscle tissue, time course of contraction of said muscle tissue, force of contraction of said muscle tissue, action potential of said muscle tissue, and time course of the action potential of said muscle tissue.

The time to paralysis of muscle tissue may be measured e.g. in seconds or minutes. According to subvariants, the time to paralysis may be determined based on the muscle contraction distance (paralysis being achieved once the contraction distance is equal to 0) or on the muscle twitch frequency (paralysis being achieved once the twitch frequency is equal to 0). The contraction distance may e.g. be measured in centimeters or millimeters. The time to paralysis may be defined as the period that passed to attain half maximum twitch. This is strictly dependent on the neurotoxin concentration. Methods for determining variation in the contraction rate of the muscle tissue, or is the variation in the contraction of the muscle tissue, or is the variation in the force of contraction of the muscle tissue are are known in the art, and are for example disclosed in EP 1 597 584 B1. An action potential may, for example, be detected by using electromyography.

In certain embodiments of the first aspect of the present invention, said inhibition is determined by indirectly determining the activity of said muscle tissue.

In the context of the present invention, the term term "indirectly determining the activity of said muscle tissue" refers to methods involving reading out parameters resulting from the activity of a muscle tissue.

In another embodiment, the activity is determined by determining one or more of the following parameters: LD₅₀ value for a population of said animal, movement of said animal, time to paralysis of said animal, muscle-driven change of color in animals with chromatophore cells.

The determination of LD50 values in an animal according to the methods of the present invention can be performed in analogy to the mouse LD₅₀ assay (see Boroff and Fleck, J. Bacteriol. 92 (1966) 1580-1581). Parameters relating to movement of animals may be, for example, distance, speed or time-to-paralysis of lateral movement of the animal, or distance (e.g. angle), frequency, speed of movement or time-to-paralysis of parts of an animal (e.g. of the tails of tadpoles), in-current and an ex-current siphoning activity of tunicates, the speed and frequence of color change in Cephalopoda, or the like.

In certain embodiments of the first aspect of the present invention, the biological activity is the inhibition of secretory activity.

In the context of the present invention, the term "inhibition of secretory activity" refers to the property of clostridial neurotoxins to inhibit activation of cholinergically controlled secretion, including sweating, lacrimation, glandular secretion, or mucus production

In certain embodiments of the present invention, the neurotoxin referred to herein is a neurotoxin of *C. botulinum* and is of serotype A, B, C, D, E, F or G or is a biologically active derivative thereof, wherein said derivative may be a genetically modified neurotoxin such as a mutant comprising a deletion of one or more amino acids, an addition of one or more amino acids and/or a substitution of one or more amino acids. Preferably, said deleted or added amino acids are consecutive amino acids. According to the teaching of the present invention, any number of amino acids may be added or deleted, as long as the neurotoxin is biologically active. For example, 1, 2, 3, 4, 5, up to 10, up to 15, up to 25, up to 50, up to 100, up to 200, up to 400, up to 500 amino acids or even more amino acids may be added or deleted. In certain aspects, the present invention refers to neurotoxins, with an addition of more than 500 amino acids, such as for example up to 600 or up to 800 additional amino acids, or even more additional amino acids. Accordingly, a derivative of the neurotoxin may be a biologically active fragment of a naturally occurring neurotoxin. This neurotoxin fragment may contain an N-terminal, C-terminal and/or one or more internal deletion(s). "Biologically active", as used in this context, means, said derivative is taken up into the nerve cell and is capable of denervating said nerve from the muscle or gland, to which it is connected. Moreover, said clostridial neurotoxin may be associated with complexing proteins or may be free of complexing proteins (see below).

In the context of the present invention, the term "botulinum neurotoxin" refers to neurotoxins obtainable from *Clostridium botulinum.* Currently, seven serologically distinct types, designated serotypes A, B, C, D, E, F and G, are known, including certain subtypes (e.g. A1, A2, and A3). In *Clostridium botulinum,* these neurotoxins are formed as protein complexes comprising a neurotoxic component and at least another non-toxic protein. Thus, the term "botulinum neurotoxin serotype A", as used herein, refers to the botulinum neurotoxin complex, such as the 450 kDa and the 900 kDa complexes which are e.g. obtainable from cultures of *Clostridium botulinum.* Preparations comprising such complexes are commercially available, e.g. from Ipsen Ltd. (Dysport®) or Allergan Inc. (Botox®).

Moreover, the term botulinum neurotoxin also refers to botulinum neurotoxin which is free of complexing proteins and which has a molecular weight of approximately 150kDa. Thus, in the context of the present invention, the term "isolated neurotoxic component of botulinum neurotoxin serotyp ..." refers to the neuroxic component of the corresponding botulinum neurotoxin serotype complex free of complexing proteins. This neurotoxic component is a two-chain polypeptide with a 100 kDa heavy chain joined by a disulfide bond to a 50 kDa light chain. The heavy chain is responsible for entry into the neuronal cell, while the light chain comprises an endopeptidase activity responsible for cleaving one or more proteins that is/are part of the so-called SNARE-complex involved in the process resulting in the release of neurotransmitter into the synaptic cleft. A high purity neurotoxic component, free of any other botulinum protein is e.g. available from Merz Pharmaceuticals GmbH, Frankfurt (Xeomin^{®}).

The term "other isolated fragment of any said botulinum neurotoxin .... where such fragment has neurotoxic activity" refers to a neurotoxin complex that lacks certain of the non-toxic proteins of a botulinum neurotoxin as defined above, or certain parts of the neurotoxic component as defined above comprised therein, while maintaining the neurotoxic activity. Correspondingly, the term "other isolated fragment of any said ... neurotoxic component, where such fragment has neurotoxic activity" refers to a neurotoxin component that lacks certain certain parts of the neurotoxic component of a botulinum neurotoxin as defined above, while maintaining the neurotoxic activity. Methods for making or identifying such fragments, and methods for identifying whether such fragments maintain neurotoxic activity, are well known to anyone of ordinary skill in the art.

In the context of the present invention, the term "derivative" and the term "variant or synthethic analogue" refer to a neurotoxin complex or neurotoxic component that is a chemically, enzymatically or genetically modified derivative of a neurotoxin as defined herein, including chemically or genetically modified neurotoxin from C. botulinum. A chemically modified derivative may be one that is modified by pyruvation, phosphorylation, sulfatation, lipidation, pegylation, glycosylation and/or the chemical addition of an amino acid or a polypeptide comprising between 2 and about 100 amino acids . An enzymatically modified derivative is one that is modified by the activity of enzymes, such as endo- or exoproteolytic enzymes. As pointed out above, a genetically modified derivative is one that has been modified by deletion or substitution of one or more amino acids contained in, or by addition of one or more amino acids (including polypeptides comprising between 2 and about 100 amino acids) to, the proteins of said neurotoxin or a neurotoxic component thereof. Methods for making such chemically or genetically modified derivatives, and methods for identifying whether such derivatives maintain neurotoxic activity, are well known to anyone of ordinary skill in the art.

In a particular embodiment, said clostridial neurotoxin is selected from the group of botulinum neurotoxin serotype A, the isolated neurotoxic component of botulinum neurotoxin serotype A, an isolated fragment of botulinum neurotoxin serotype A or of the neurotoxic component of botulinum neurotoxin serotype A, where such fragment has neurotoxic activity, and any variant or synthetic analogue of botulinum neurotoxin serotype A or of the neurotoxic component thereof, where such variant or analogue has neurotoxic activity. The term "fragment" relates to a neurotoxin lacking 1, 2, 3, 4, 5, up to 10, up to 15, up to 25, up to 50, up to 100, up to 200, up to 400, up to 500 amino acids or even more amino acids. Preferably, said amino acids are consecutive amino acids.

Particularly, said clostridial neurotoxin is the isolated neurotoxic component of botulinum neurotoxin of serotype A, which is available from Merz Pharmaceuticals GmbH, Frankfurt (Xeomin^{®}).

In certain embodiments of the first aspect of the present invention, said clostridial neurotoxin preparation is for pharmaceutical and/or aesthetic use.

In a particular embodiment of the method according to the first aspect of the invention, said method comprises the step of comparing the biological activity of a clostridial neurotoxin preparation for pharmaceutical and/or aesthetic use with the biological activity of a standard preparation of a reference clostridial neurotoxin in an an animal belonging (i) to the phylum: Mollusca; (ii) to a subphylum taken from the list of: Tunicata and Hexapoda (at all stages of development, including nymphae, larvae, pupae and imagines); or (iii) to a class taken from the list of: Amphibia (at all stages of development, including tadpoles and adults) and Reptilia, , wherein the clostridial neurotoxin in said clostridial neurotoxin preparation is selected from the list of: botulinum neurotoxin serotype A, the isolated neurotoxic component of botulinum neurotoxin serotype A, any other isolated fragment of botulinum neurotoxin serotype A or of the neurotoxic component of botulinum neurotoxin serotype A, where such fragment has neurotoxic activity, and any variant or synthetic analogue of botulinum neurotoxin serotype A or of the neurotoxic component thereof, where such variant or analogue has neurotoxic activity.

In certain embodiments of the present invention, the clostridial neurotoxin in the clostridial neurotoxin preparation is produced in a fermentation process.

In particular embodiments the clostridial neurotoxin preparation is produced by a fermentation process using *Clostridium botulinum* strains. Methods for the fermentation process using *Clostridium botulinum* strains are well known in the art (see, for example, Siegel and Metzger, Appl. Environ. Microbiol. 38 (1979) 606-611; Siegel and Metzger, Appl. Environ. Microbiol. 40 (1980) 1023-1026).

In particular embodiments, the clostridial neurotoxin preparation is produced in heterologous cells, i.e. it is produced recombinantly by expressing nucleic acid sequences encoding a neurotoxin in an appropriate host cells. Methods for the recombinant expression of clostridial neurotoxin are well known in the art (see, for example, WO 2006/076902 or EP 1 206 554). An example of a heterologous cell is *E. coli.*

In certain embodiments of the present invention, the clostridial neurotoxin in said clostridial neurotoxin preparation and the reference clostridial neurotoxin belong to the same serotype.

In particular embodiments, the clostridial neurotoxin in said clostridial neurotoxin preparation and the reference clostridial neurotoxin are identical.

In this context, the terms "belong to the same serotype" and "identical" refer to the nature of the active neurotoxic agent present in the clostridial neurotoxin preparation and the reference clostridial neurotoxin. For example, when the clostridial neurotoxin preparation contains the isolated neurotoxic component (i.e. without any complexing proteins) of botulinum neurotoxin serotype A, and the reference clostridial neurotoxin is botulinum neurotoxin serotype A (i.e. the full complex), the clostridial neurotoxin in said clostridial neurotoxin preparation and the reference clostridial neurotoxin belong to the same serotype, but are not identical. When the clostridial neurotoxin preparation contains the isolated neurotoxic component (i.e. without any complexing proteins) of botulinum neurotoxin serotype A, and the reference clostridial neurotoxin is the isolated neurotoxic component of botulinum neurotoxin serotype A as well, the clostridial neurotoxin in said clostridial neurotoxin preparation and the reference clostridial neurotoxin are identical (even though the clostridial neurotoxin preparation and the preparation containing the reference clostridial neurotoxin may differ in the amount and concentration of the respective neurotoxin and/or the presence of additional ingredients such as buffers, excipients etc.).

In another aspect, the present invention relates to an in vivo method for determining the biological activity of a clostridial neurotoxin preparation, comprising the steps of (a) contacting an animal with a sample of the clostridial neurotoxin preparation; and (b) comparing the biological effect of the sample with the biological effect of a reference sample; wherein said animal is a Mollusca.

The term "in vivo" as used herein refers to animals, which are alive. In other words, the method of the present invention involves contacting a living animal with a sample and observing the effect that the sample has on the living animal. The term "contacting" as used herein means exposing the animal or parts of the animal to the sample. One way of exposing the animal to the sample is to incubate (bathe) the animal in the sample. To this end, the animal may be kept e.g. in an aquarium, e.g. filled with sea water (taken from the sea) or in fresh water (taken from a river or lake). The skilled person will realize that the choice of water depends on the natural environment of the animal. In order to expose the animal to the sample, a suitable concentration of the sample may be added to the aquarium. An alternative way to contact the animal with the sample, is to inject the sample directly into muscular tissue of the animal.

The skilled person will recognize that a minimal incubation period may be required to observe an effect of the sample comprising the clostridial neurotoxin on the animal. Although incubation periods may be different from species to species, the skilled person can easily establish suitable conditions, which will also depend on the final concentration of the neurotoxin.

The term "effect" as used herein refers to activities of the animal. Examples of such activities are the opening and/or closing of the shell or movement activity of the animal. In one embodiment, the effect of the sample comprising a clostridial neurotoxin is determined by an LD₅₀ assay. In this case, the term "effect" means dying of the animal.

According to the present invention's teaching, the effect of the sample containing the clostridial neurotoxin is compared to the effect of a reference sample. The term "reference sample" refers to a preparation with a known quantity of neurotoxin. In one embodiment, the effect of the sample is compared to a standard dose-response curve established by measuring the effect of several reference samples, each containing a different amount of the reference neurotoxin. In certain embodiments, the quantity of the reference sample (i.e. the amount per standard unit, e.g. mg/ml, or mg/kg of body weight) is plotted on the X axis and the response is plotted on the Y axis. Commonly, it is the logarithm of the quantity that is plotted on the X axis, and in such cases the dose-response curve is typically sigmoidal, with the steepest portion in the middle, indicating the half-maximal response. On the Y axis, the response is typically expressed as percentage of the maximal response observed, so that the quantity causing the half-maximal response is the quantity causing a 50% response rate, e.g. a 50% lethality (LD₅₀), 50% inhibition (IC₅₀), or 50% efficacy (EC₅₀).

In one embodiment, the biological effect of the sample and the biological effect of the reference sample are determined in the same animal species.

In one embodiment, the biological activity is determined by determining one or more of the following parameters: time to paralysis of muscle tissue, contraction rate of muscle tissue, contraction distance of muscle tissue, time course of contraction of muscle tissue, force of contraction of muscle tissue, action potential of muscle tissue, and time course of the action potential of muscle tissue.

In one embodiment, the biological activity is determined by performing an LD₅₀ for a population of said animal.

In one embodiment, the animal is a Mollusca selected from the group consisting of Aplacophora, Bivalvia, Caudofoveata, Cephalopoda, Gastropoda, Monoplacophora, Polyplacophora and Scaphopoda.

In one embodiment, the animal is a Bivalvia and the biological activity is determined by determining the muscle activity involved in closing the Bivalvian shell.

In one embodiment, the muscle activity is determined by measuring the force required to open the shell. The force may, for example, be determined by using a force gauge, such as a digital or mechanical force gauge.

### EXAMPLES

### Example 1: Determination of LD₅₀ in Blattodea

A fixed amount of different dilutions from a dilution series of a reference botulinum neurotoxin serotype A with known biological activity (expressed as mouse units) is injected into the abdominal part of *Blatella germanica* (10 animals for each dilution), and the LD₅₀ value is determined essentially as well known from the mouse LD₅₀ assay.

The same experiment is repeated with a dilution series of the clostridium neurotoxin preparation in otherwise identical fashion. The amount of clostridium neurotoxin preparation necessary to achieve LD₅₀ can then be correlated to the corresponding amounf of reference botulinum neurotoxin serotype A with known biological activity.

### Example 2: Determination of LD₅₀ for Water Animals

The experiment is performed as described in Example 1, with the exception that water animals, such as *Aplysia californica*, sea squirts, frog tadpoles etc. are used, and that the neurotoxin solutions are not injected but added to the water reservoir harboring the animals.

### Example 3: Determination of IC₅₀ for Water Animals

The experiment is performed essentially as described in Example 2, with the exception that non-lethal amounts of the neurotoxin dilutions are being added to the water reservoirs, and that instead of an LD₅₀ value an IC₅₀ value, representing the amount at which 50% of the animals have been paralyzed, or where a certain movement of the animals or of a part thereof has been paralyzed or inhibited in 50% of the animals, is determined and used for correlating the biological activity of the clostridium neurotoxin preparation with the known biological activity of the reference botulinum neurotoxin serotype A.

### Example 4: Determination of Inhibition of Muscle Activity in Mytilis Edulis

In mussels of the class "Bivalvia", the two-part shells are held tightly together by an adductor muscle. Methods and apparatuses for determining the forces required for opening the mussels have been described in the literature (see, for example, Lavoie, Biol Bull 111 (1956) 114-122). Similar types of experiments as described in Lavoie can be used for deterimining the inhibition of muscle activity by clostridial neurotoxins.

In a first experiment, a number of *Mytilis edulis*, each having a length of 5 cm ± 0.25 cm, are kept in an experimental setting as shown in El-Shenawy et al. ( Pak. J. Applied Sci. 3 (2003) 687-702), and the valve movement and the maximum opening of the valves in fresh seawater is recorded. Next, each mussel is attached to an apparatus as shown in Lavoi, exerting a constant force by attaching a weight with a mass of 800 g. Mussels that open by more than 1 mm are discarded. Next, a dilution series of a reference botulinum neurotoxin serotype A with known biological activity (expressed as mouse units) is added to the aqueous environment of the mussels, and the concentration of reference botulinum neurotoxin is determined that results in 50% of the mussels having a gap of more than 90% of the average maximum opening . Such a concentration of the reference botulinum neurotoxin is then the LD₅₀ value (if the mussels are killed so that the shells open), or an IC₅₀ value (if the mussels survive, but the adductor muscle activity is inhibited).

By performing the same experiment using a dilutions series of the clostridium neurotoxin preparation in otherwise identical fashion, the biological activity of the clostridium neurotoxin preparation can be correlated with the known biological activity of the reference botulinum neurotoxin serotype A.

Alternatively, the same assay format can be used in order to measure the force required to open the shells of *Mytilis edulis* up to more than 90% of the average maximum opening , when a given amount of the reference botulinum neurotoxin serotype A is added to the test system. By using a series of amount of the reference botulinum neurotoxin serotype A, a dose-response (i.e. inhibition) curve can be established, and a linear range can be identified. Then, one or more samples of the clostridium neurotoxin preparation to be tested can be added to the assay system, and the respective forces required to open the shells of *Mytilis edulis* up to more than 90% of the average maximum opening can be determined. By correlating the forces required with the dose-response curve for the reference botulinum neurotoxin serotype A, the biological activity of the clostridium neurotoxin preparation can be determined with respect to the known biological activity of the reference botulinum neurotoxin serotype A.

### Example 5: Determination of Inhibition of Color Change in Cephalopoda

Cephalopoda are able to achieve rapid changes of the colors of their skin as a means of camouflage. The color change is brought about by chromatophore organs, which are cytoelastic sacs of pigment with radial muscles. These muscles are innervated by motoneurons. Under physiological conditions, color change is a rapid process. For example, the color change in *Octopus vulgaris* resulting from getting in contact with a diver was determined to be completed in about 2 seconds (see Hanlon, Current Biology 17 (2007) 400-R404).

For the assay a Cephalopoda such as *Octopus vulgaris* or *Sepia officinalis* is placed in an aquarium. By applying an external stimulus, such as the appearance of a silhouette simulating a predatory enemy for the Cephalopoda, or by placing the aquarium on a monitor surface and changing the color of the picture displayed by the monitor, the Cephalopoda is caused to perform its color change, and the time for achieving such changes under physiological conditions is determined.

Next, the experiment is repeated several times using increasing (non-lethal) amounts of a reference botulinum neurotoxin serotype A being added to the water in the aquarium. Preferably, separate experiments are performed for each data point. The corresponding times for achieving the color changes in the presence of botulinum toxin are monitored. Preferably, a concentration range is identified where a linear relationship between the amounts being added and the corresponding times for achieving the color change exists.

Finally, another animal in a separate aquarium is treated with a defined amount of the clostridium neurotoxin preparation to be tested (preferably a botulinum neurotoxin serotype A as well, most preferably of the same type as the reference botulinum neurotoxin serotype A), and the time for achieving the color change in the presence of the clostridium neurotoxin preparation is monitored. By comparing that time with the set of data of the experiments with the reference botulinum neurotoxin serotype A or the extrapolation therefrom, the biological activity of the clostridium neurotoxin preparation, expressed in terms of the known biological activity of the reference, is possible.

## Claims

1. An in vivo method for determining the biological activity of a clostridial neurotoxin preparation, comprising the steps of:
(a) contacting an animal with a sample of said clostridial neurotoxin preparation; and
(b) comparing the biological effect of the sample with the biological effect of a reference sample;
wherein said animal is a Mollusca.

2. The method of claim 1, wherein the biological activity is determined by determining one or more of the following parameters: time to paralysis of muscle tissue, contraction rate of muscle tissue, contraction distance of muscle tissue, time course of contraction of muscle tissue, force of contraction of muscle tissue, action potential of muscle tissue, and time course of the action potential of muscle tissue.

3. The method of claim 1 or 2, wherein said biological activity is determined by determining an LD₅₀ for a population of said animal or by measuring motility of said animal.

4. The method of any one of claims 1 to 3, wherein the animal is a Mollusca selected from the group consisting of Aplacophora, Bivalvia, Caudofoveata, Cephalopoda, Gastropoda, Monoplacophora, Polyplacophora and Scaphopoda.

5. The method of claim 4, wherein the animal is Bivalvia and wherein the biological activity is determined by determining the muscle activity involved in closing the Bivalvian shell.

6. The method of claim 5, wherein the muscle activity is determined by measuring the force required to open the shell.

7. The method of claim 6, wherein the force is determined by using a force gauge.

8. The method of any one of claims 1 to 7, wherein said clostridial neurotoxin is of serotype A, B, C, D, E, F or G or is a biologically active derivative thereof.
